# EUROPEAN PATENT SPECIFICATION

(11) **EP 4 434 460 B1**
(45) Date of publication and mention of the grant of the patent: **04.03.2026**
(21) Application number: 23163690.3
(22) Date of filing: 23.03.2023
(51) Int. Cl.: A61B 5/20

(54) **SINGLE-USE DISPOSABLE CONTAINER WITH ANALYTICAL SENSOR**
EINWEGBEHÄLTER MIT ANALYTISCHEM SENSOR ZUR EINMALIGEN VERWENDUNG
RÉCIPIENT JETABLE À USAGE UNIQUE AVEC CAPTEUR ANALYTIQUE

(43) Date of publication of application: 25.09.2024
(73) Proprietor: Wellspect AB, 431 21 Mölndal (SE)
(72) Inventor: BERTHUEL, Marie, 38610 Gieres (FR); CLAESSON, Henrik, 437 38 Lindome (SE); D ACCRISCIO, Florian, 38190 Les Adrets (FR); HAMMOND, Jules, 38610 Gieres (FR); LOVMAR, Martin, 431 69 Mölndal (SE); POINTEAUX, Léo, 38240 Meylan (FR); SERGEJEVS, Aleksandrs, 38610 Gières (FR); UTAS, Jan, 434 95 Kungsbacka (SE)
(74) Representative: Aldridge, Henry Alexander

(56) References cited:
- WO-A1-2018/217818
- WO-A1-2018/223090
- US-A1- 2005 288 608
- US-A1- 2010 064 797
- US-A1- 2023 018 531

## Description

### TECHNICAL FIELD OF THE INVENTION

The present invention relates to an analytical sensor device comprising a biofuel cell powered sensor arranged in a disposable container.

### BACKGROUND OF THE INVENTION

In the field of urology it is common to perform different types of measurements to determine the health of a patient from which the urine sample was obtained. For instance, a urine sample can be analyzed with a dipstick, pH sensor or with more sophisticated equipment such as a spectrometer to determine the concentration of different urine constituents, such as minerals, proteins and sugars, or the concentration of other substances, such as drugs. It is also possible to detect the presence of different types of bacteria, such as *E. coli* and *Staphylococcus* spp, which may indicate that the patient suffers from a bacterial infection.

Furthermore, it is often desirable to perform other types or measurements to determine e.g. the total amount of urine discharged by a patient and, perhaps more importantly, to determine the flow rate at which urine is discharged by a patient. Urine flow rate measurements, often referred to as uroflowmetry, are important as too low flow rates could serve as a preliminary indicator of poor function in the bladder, strictures, an enlarged prostate etc.

A simple method for getting a rough estimate of the urine flow rate is to simply measure the time it takes for a patient to discharge a certain volume of urine and calculate the ratio of the urine volume and the measured time. While this gives some estimate of the average urine flow rate, it is rarely accurate enough to establish whether the urine flow appears to be healthy or not. For instance, the urine flow may vary rapidly over time, and it has been understood that especially the peak urine flow rate, often referred to as Qₘₐₓ is one of the most important factors to consider in a preliminary uroflowmetry examination.

As the time it takes for a patient to discharge a certain volume of urine cannot be used to determine Qₘₐₓ, or indeed any instantaneous urine flow rate, various uroflowmetry devices have been developed to make the measurements more accurate and reliable.

For instance, in German patent application number 20141008760 a uroflowmetry funnel is presented which directs urine towards an impeller which rotates at a rotational speed which depends on the urine flow rate. By measuring the rotational speed of the impeller with a sensor, the instantaneous urine flow rate can be accurately acquired. As another example, in US application number 11,124,385 a cup is placed onto a pressure sensor. When urine is discharged into the cup, the pressure measured by the pressure sensor increases and the instantaneous rate at which the pressure increases is proportional to rate at which urine enters the cup.

However, a drawback with existing solutions is that the devices for uroflowmetry often are bulky, consists of multiple parts (e.g. an externally powered pressure sensor and a cup which should be placed at the pressure sensor), difficult to clean and/or sterilize between uses for multiple use devices or in general too complicated to be manufactured as disposable, single use, products. Accordingly, uroflowmetry measurements are still complicated to perform, often requiring patients to travel to a nearby hospital or clinic to perform uroflowmetric measurements with specialized equipment. As a result, uroflowmetric measurements are often underutilized despite offering a good first indicator whether a patient should undergo further urologic examination.

US2005288608A1 discloses an ambulatory device for measuring urine flow comprises a portable hand-held container and a handgrip mounted thereto. A flow-measuring device is located in the container, and a means for collecting the data measured by the flow-measuring device is also provided. The parameters of the urine flow in the container are measured by the flow-measuring device and are processed by the aforementioned means for collecting data. The handgrip is pivotally mounted to the container by a double-pivot mechanism for maintaining the container substantially vertical in a number of positions of the handgrip. The flow-measuring device includes a sensor that measures a displacement of an air column related to a variation of pressure due to a variation of a urine level in the container.

WO2018223090A1 discloses a sensing platform for sensing and analysis of biofluids, particularly well-suited for sensing and analysis of sweat. Systems of the invention allows for sensitive and selective detection of a range of analytes in sweat including metabolites, electrolytes and biomarkers. Systems of the invention provide a noninvasive and accurate means for quantitative characterization of important sweat characteristics including sweat volume, sweat loss and sweat rate. Systems of the invention are compatible with materials and device geometries for important class of conformal tissue mounted electronic devices, including epidermal electronic devices.

US2010064797A1 discloses an apparatus including: a container that receives urine; and a urine amount measuring device that measures the weight of the urine received by the container; wherein the urine amount measuring device has: a mounting plate, which is a plate on which the container is mounted; a measuring portion that measures the weight of the container mounted on the mounting plate multiple times at given time intervals; and an output portion that outputs a result of the measurement performed by the measuring portion, and the apparatus has a fixing structure that is situated in at least a bottom portion of the container and a mounting face of the mounting plate and that detachably fixes the container on the mounting plate.

US2023018531A1 discloses a device includes: housing having top surface, bottom surface, and at least one side surface connected between top surface and bottom surface; a load cell positioned within housing; flexible section positioned within at least one of bottom surface or top surface of housing; rigid section positioned within flexible section, rigid section having opening surrounded by rigid material; rigid support having first end, second end, and center section; wherein first end of rigid support is positioned within housing and coupled to load cell; and wherein center section of rigid support passes through opening in rigid section positioned within flexible section, wherein center section of rigid support contacts rigid material of rigid section surrounding opening, wherein second end of rigid support is outside of housing.

WO2018217818A1 discloses a printed circuit board device includes: a first capacitive sensor configured to measure a first capacitance within a contained volume having known dimensions, wherein the first capacitance changes as a substance is received into the contained volume; a second capacitive sensor having a plurality of trigger points at a plurality of corresponding known heights within the contained volume, the second capacitive sensor configured to detect when the substance received into the contained volume has reached each of the corresponding known heights within the contained volume; and wherein at least one of a level of the substance within the contained volume, a volume of the substance within the contained volume, or a flow rate of the substance into the contained volume is determined based on data from the first capacitive sensor and the second capacitive sensor.

### SUMMARY OF THE INVENTION

In view of the drawbacks of existing solutions there is a need for an improved device and method for performing analytical fluid measurements, and especially uroflowmetric medical measurements. The purpose of the present invention is to provide such an improved device and method, which overcomes at least some of the drawbacks mentioned in the above.

The invention is set out in the claims.

According to a first aspect of the invention there is provided a disposable analytical sensor device comprising a disposable container comprising an opening in fluid communication with an inside of the disposable container and at least one sensor arranged in fluid communication with the inside of said disposable container, the at least one sensor being configured to measure at least one property of a fluid present in the disposable container. The disposable analytical sensor device further comprises an energy source arranged on said disposable container and connected to said at least one sensor, wherein the energy source comprises a biofuel cell arranged to provide power using a fluid.

The energy source can be produced in any form such as circle, square, rectangle, or pentagon for instance. In some implementations, the energy source comprises two or more biofuel cell arranged on said disposable container.

The fluid may be a liquid. In some implementations, the fluid is liquid urine discharged by a patient directly into the disposable container or urine poured into the analytical sensor device from a urine sample container. In such implementations, the analytical sensor device may be referred to as a "medical sensor device" as it can be used for medical investigation purposes. In some examples presented herein, the fluid will be assumed to be urine, however the same features and associated benefits are still applicable if the fluid is some other liquid besides urine. For example, it is envisaged that the fluid may be saliva or blood from a patient. It is also envisaged that the fluid is not a bodily fluid at all and that the analytical sensor device is used for non-medical purposes. For example, the fluid is a water, mud or soil. In one example implementation, the analytical sensor device is used to test the properties of water flowing e.g. from a tap or taken as sample from a body of water. The analytical sensor device could e.g. measure the flow rate of water leaving a tap and/or determine one or more properties of the water (such as the pH-level or the presence of bacteria).

The at least one fluid/urine property measured with the at least one sensor may be a physical property of the fluid/urine entering the container, such as the fluid/urine temperature, the fluid/urine flow rate, the fluid/urine volume, the fluid/urine density, the fluid/urine weight, the fluid/urine viscosity and the like. The at least one fluid/urine property may additionally or alternatively be a chemical property of the fluid/urine, such as the pH-level of the fluid/urine, or presence and/or concentration of a fluid/urine constituent or other foreign substance, including but not limited to blood, chloride, sodium potassium, creatinine or other dissolved ions and inorganic or organic compounds such as drugs or secondary metabolites of drugs. The fluid/urine property may be a property measured at a single instance in time, or the fluid/urine property may be a property that is a function of time, for example the fluid/urine flow rate as a function of time.

The present invention is at least partially based on the understanding that it may be highly advantageous to non-invasively measure a fluid/urine property with a disposable, single-use, and simple product. For instance, for urine measurements, this helps avoiding unnecessary surgery and enables measurements to be performed more often. This is achieved by providing a disposable container provided with at least one (preferably disposable) sensor and a (preferably disposable) biofuel cell powered energy source resulting in a ready-to-use product which is intuitive, user-friendly, simple to manufacture and disposable. Similarly, the same product may be advantageous to use when analyzing other fluids besides urine, such as when analyzing a water sample or soil sample.

With the term "disposable" it is, in the context of the present application, meant an analytical sensor device which can be disposed after a single and/or short time use, in an environmentally friendly way. The container, and also the sensor and biofuel cell, of the disposable analytical sensor device is preferably made of a bio-based and/or biodegradable material. Put differently, the materials used in the disposable analytical sensor device are preferably to a large extent, and preferably totally, bio-based and/or biodegradable and/or compostable. By a biodegradable material is here meant an organic material which breaks down by microorganisms, such as bacteria and fungi, in a microbial process, and preferably under environmental exposure, e.g. making the material compostable. A biodegradable material may also be biodegradable in accordance with any of the standards ASTM D6868 and EN 13432, and may also be compostable in accordance with the standard ASTM D6400.

Any electrical circuits of the analytical sensor device (e.g. the sensor, controller/CPU and any electrical connections therebetween) may be produced as thin film electronics which can be attached to the inside of the container and/or outside of the container, or integrated into the container. Preferably, at least the sensor is arranged to be in direct contact with the fluid/urine added to the inside of the container. This may be achieved by arranging the sensor inside the container or integrating the sensor in the container and providing a fluid channel leading to the sensor from the inside of the container.

The electrical components may be arranged on a substrate also referred to as an electronic platform. The electronic platform(s) may be produced in any form such as circle, square, rectangle, or pentagon for instance. Preferably, at least some parts of the electronics are organic, for example the circuits may comprise conductive polymers and/or other carbonbased conductors. In some implementations, the electronic platform is composed of the biofuel cell, a controller/CPU and a fluid reservoir and the electronic platform is (or at least is mounted on) a sticker. The sticker can advantageously be sticked on any part of the disposable container and connected to the sensor.

In some implementations, the at least one sensor is configured to measure the flow rate of the fluid/urine entering the disposable container. The sensor may be configured to measure the flow rate directly or indirectly. For example, the sensor may measure the flow rate indirectly by measuring a change in fluid pressure or an electrical property between two terminals as will be described in further detail below.

To this end, the analytical sensor device may be used to perform uroflowmetric measurements which has previously been complicated measurements requiring large, specialized equipment commonly only available at hospitals. Uroflowmetric measurements may serve as an important first indicator to whether further medical examination is required. With the analytical sensor device, these measurements are easy to perform allowing the patient to perform the measurements on their own, e.g. at home. If the uroflowmetric measurements indicate e.g. that the maximum urine flow rate is too low, this may be a sign of an enlarged prostate for male patients allowing the patient to seek further medical examination. Similarly, if the flow rate is too low this may be a sign of underactive bladder (UAB) for both male and female patients.

The analytical sensor device is also cheap to manufacture in large quantities, making it affordable and suitable for large scale screening. Thus, many more patients will be able to perform uroflowmetric measurements, which increases the chance of providing patients with an early warning and to seek further urologic examination at an early stage.

In some implementations, the at least one sensor comprises a pressure sensor, configured to measure the fluid pressure of the fluid/urine inside the disposable container at different points in time wherein a difference in pressure between the different points in time indicate the flow rate of the fluid/urine.

In this way, an accurate measurement of the instantaneous fluid/urine flow rate can easily be acquired using a simple fluid pressure sensor. By measuring the fluid pressure at different points in time it is for example possible to determine the changes in flow rate during a patient's voiding process in uroflowmetric applications.

In some implementations, the at least one sensor is two or more pressure sensors, arranged to measure the fluid pressure at two or more different locations inside the disposable container.

With such an arrangement, the accuracy of the pressure measurements is further enhanced as e.g. the sensor data from all sensors can be combined (e.g. averaged) to form a more reliable and stable measurement. Additionally, two or more pressure sensors arranged at different locations enables any tilt of the analytical sensor device during the measurements to be compensated for, as will be described in detail more in the below. In some implementations, the at least one sensor is an electrical sensor connected to at least two electrical terminals arranged with a separation distance inside the disposable container. Wherein the electrical sensor is configured to measure an electrical property of the media between the two terminals at different points in time. A difference in the electrical property between the different points in time indicates the flow rate of fluid/urine entering the container.

As a fluid enters the container, the fluid/urine level inside the container will rise, effectively changing one or more electrical properties of the space between the at least two terminals. This means that the measured electrical property will be linked to the amount of fluid/urine inside the container and that any change in electrical property will be linked to a fluid/urine flow rate. For instance, the terminals are arranged at inner side walls of the container, or the terminals are arranged with one terminal at the bottom and one terminal at the side wall. Preferably, multiple pairs of terminals are arranged at intervals along a height direction from the bottom to the open top. As the fluid/urine reaches each pair there will be a drastic change in the electrical property measured between the pair, making it easy to determine when the amount of fluid/urine has reached a certain level (volume).

The measured electrical property is at least one of capacitance, inductance, conductivity and resistance (such as charge transfer resistance, Warburg resistance, etc.) between the two terminals.

In some implementations, the at least one sensor is configured to measure at least one of a concentration of a fluid/urine constituent, such as urea, sugars (e.g. glucose), bacteria, minerals, or proteins, a pH level of the fluid/urine, a temperature of the fluid/urine and a foreign substance that can be present in the fluid/urine, such as drugs or secondary metabolites of drugs.
It is understood that the energy source can power many types of sensors and that the property which is sensed could be properties other than those linked to the fluid/urine flow rate. For example, the sensor could measure the pH-level or be an electronic dipstick measuring the concentration of glucose in the fluid/urine. It is also envisaged that the sensor measures the presence or concentration of bacteria, such as *E. coli,* in the fluid/urine.

In some implementations the analytical sensor device further comprises a controller or CPU which is also powered by the energy source. The controller or CPU may be configured to obtain sensor data from the sensor and control, based on the sensor data, a wireless transmitter or a presentation unit for emitting light, sound or displaying a graphical symbol.

That is, the controller may enable the analytical sensor device to present a visual or acoustic indicator via the presentation unit which can be perceived by the user, a patient or the patient's assistant (e.g. a medical professional). In some embodiments, the presentation displays a light, symbol or graphical message, or emits a sound, e.g. if the sensor data indicates that a maximum fluid/urine flow rate is below a predetermined threshold.

In some implementations, the analytical sensor device further comprises a wireless transmitter (e.g. an antenna) being powered by the energy source and configured to wirelessly transmit, to an external device, measurement information based on sensor data collected by the at least one sensor. For example, the antenna is printed on a sticker, which is polymerbased or preferably paper-based, that can be stuck onto the outside of the container and connected to other electrical components. Alternatively, the antenna is printed directly on the outside of the container. The antenna could be arranged anywhere on the outside of the container, such as on the curved wall or in a cavity underneath the container, or the antenna could be integrated within the container. The antenna can be shaped in any form such as circle, square, rectangle, or pentagon for instance.

The measurement information may be equal to the sensor data. Alternatively, the measurement information is a processed version of the sensor data (e.g. a sensed fluid pressure or electrical property converted to a measured flow rate) or the result of an analysis of the sensor data or the measurement information (e.g. determined maximum fluid/urine flow rate, average flow rate or total amount of fluid/urine added to the container). To this end, it is not necessary for the analytical sensor device to comprise a presentation unit as the measurement information can be transmitted to an external device (e.g. a smartphone) which can present the measurement information.

In some implementations, the biofuel cell is arranged inside the disposable container, preferably at a bottom portion of the disposable container. In this way, the fluid/urine added to the container will contact the biofuel cell and activate it. In other words, the fluid/urine entering the container will power the biofuel cell meaning that measurements will start automatically when the fluid/urine is added.

In some implementations, the analytical sensor device comprises a fluid reservoir containing an activation fluid, arranged on the inside of the disposable container, wherein said fluid reservoir is configured to be opened to release the activation fluid such that the activation fluid comes into contact with, and activates, the biofuel cell.

For urine measurements (as well as other fluid measurements) the activation fluid will enable the biofuel cell to be activated (i.e. in a state of being able to supply power) already before the patient discharges urine into the container. As a result, the sensor measurements can start immediately, or even before, urine enters the container, which ensures that the entire voiding process is captured in the sensor data.

In some implementations, the biofuel cell is arranged on the outside of the disposable container. In such implementations, the fluid reservoir may be arranged on or in close proximity to the biofuel cell to enable activation of the biofuel cell. The biofuel cell is arranged inside an enclosed space, the enclosed space further comprising a fluid reservoir with an activation fluid, wherein said fluid reservoir is configured to be opened to release the activation fluid inside the enclosed space such that the activation fluid comes into contact with, and activates, the biofuel cell.

In some implementations, the disposable container is made of a cellulose-based material (such as paper): Preferably, to withstand the fluid/urine, the disposable container has, on at least the inside of the disposable container, a non-cellulose-based layer such as a (hydrophobic) polymeric film or (hydrophobic) coating, a wax or a clay or a surface-modified cellulose (e.g. a chemically modified cellulose layer). Alternatively the disposable container is completely or partly made of a hydrophobized cellulose-based material (e.g. xerocellulose etc.).

Preferably, at least the inside of the container is coated with a bio-based polyethylene, bio-based wax or other type of bio-based lining. The analytical sensor device can thus be made of a cheap, disposable, recyclable and/or already recycled material. However, the non-cellulose material does not need to withstand fluid/urine for a long period of time as the measurement process only takes a few minutes whereafter the container is emptied and disposed of. To this end, it is envisaged that the container may be made entirely of a cellulose-based material, such as paper.

In some implementations, the disposable container extends along a height axis, from the bottom portion to the opening, and the disposable container has a uniform cross-sectional shape with a fixed diameter along the height axis or a cross-section with a diameter which increases or decreases along at least a portion of the height axis. It is envisaged that the change in diameter may be continuous or discrete, for example the diameter may increase/decrease abruptly. Many different shapes of the container are possible. As described in the below, certain shapes are especially beneficial and e.g. particularly well suited for female patients or male patients.

In some implementations, the inside of the disposable container is provided with an anti-slosh and/or anti-splash baffle. The baffle will reduce sloshing and splashing of fluid/urine even when fluid/urine enters the container as a highly confined stream. By mitigating splashing the analytical sensor device becomes more hygienic, as the risk of fluid/urine splashing out of the container onto e.g. a patient, the patient's assistant or the environment, is reduced. By reducing sloshing the fluid/urine level will rise more evenly in the container (with less waves and less turbulent behavior) which enables more accurate measurements, and particularly more accurate pressure measurements and/or more accurate electrical property measurements.

In some implementations, at least one dipstick is arranged on the inside of the disposable container, the dipstick being configured to provide a visible indicator in response to one or more predetermined constituents being present in the fluid/urine. The dipstick may be a regular dipstick which is not electronic but indicates visually the presence or concentration of a fluid/urine constituent or other compound. The dipstick may be arranged inside the container such that it is visible through the fluid/urine after the fluid/urine has been added to the container or after the fluid/urine has been poured out of the container. It is also envisaged that the container is provided with a transparent window, allowing the visual indicator of the dipstick to be seen from the outside of the container. As another alternative the dipstick is arranged to be pulled out of the container after it has come into contact with fluid/urine. Preferably, the dipstick is arranged to be pulled out of the container by a user without coming into contact with the fluid/urine. For example, the dipstick is attached to a string or rod which is configured so that it is not fully submerged by fluid/urine. To this end, the analytical sensor device may combine electrically powered measurements with traditional measurements, making it e.g. a very capable urologic examination tool realized as a single disposable device.

According to a preferred aspect of the invention there is provided an analytical sensor system comprising an analytical sensor device according to the first aspect of the invention with a wireless transmitter configured to transmit measurement information to an external device, and an external device configured to receive and present the measurement information. The external device may be a smartphone, smartwatch, laptop or any other computing device with e.g. a light/sound emitting device or a graphical display device for presenting information. The wireless communication is preferably enabled with a short-range, low-energy, technology such as Bluetooth (preferably Bluetooth Low Energy, BLE) or WiFi.

According to a second aspect of the invention, there is provided a method for measuring at least one property of fluid/urine, the method comprising providing an analytical sensor device according to the first aspect of the invention, discharging fluid/urine into the disposable container, and measuring the at least one fluid/urine property with the at least one sensor.

In some implementations of the second aspect, the analytical sensor device is further provided with a fluid reservoir containing an activation fluid, arranged on the inside of the disposable container or directly in contact with the biofuel cell when the biofuel cell is placed outside the disposable container, and the method further comprises the step of opening the fluid reservoir to release the activation fluid such that the activation fluid comes into contact with, and activates, the biofuel cell. This step is performed prior to discharging fluid/urine into the container. The activation fluid is any fluid suitable for activating the biofuel cell. For instance, it is possible that the activation fluid is water, or water comprising at least one additive (e.g. salt or inorganic chemical(s)) which makes the water a conductive electrolyte.

The invention according to the second aspect features the same or equivalent benefits as the invention according to the first aspect. Any functions described in relation to a method may have corresponding features in a device, and vice versa

### BRIEF DESCRIPTION OF THE DRAWINGS

The present invention will be described in more detail with reference to the appended drawings, showing currently preferred embodiments of the invention.
Figure 1 depicts a cross-sectional view of an analytical sensor device according to some implementations.
Figure 2a and 2b shows a cross-sectional view of an analytical sensor device with a fluid reservoir according to some implementations.
Figure 3a, 3b and 3c depicts cross-sectional views of different container shapes according to some implementations.
Figure 4 depicts a cross-sectional view of an analytical sensor device provided with two or more terminals for sensing an electrical property between the terminals according to some implementations.
Figure 5a and 5b depicts, in view from the top and along a cross-section respectively, an analytical device with two or more pressure sensors arranged at different locations inside the container according to some implementations.
Figure 6 depicts a cross-sectional view of an analytical sensor device provided with a baffle for reducing fluid/urine sloshing and splashing according to some implementations.
Figure 7a, 7b, 7c, 7d, 7e, 7f, 7g, 7h depicts top-views and cross-sectional views of the analytical sensor devices provided with different types of baffles according to some implementations.
Figure 8a and 8b depicts cross-sectional views of an analytical sensor device powered with an electronic platform located in the underneath cavity according to some implementations.
Figure 9a, 9b and 9c depicts bottom view and cross-sectional views of an analytical sensor provided with a wireless transmitter according to some implementations.
Figure 10 depicts a cross-sectional view of an analytical sensor device powered with an electronic platform on a sticker according to some implementations.
Figure 11 is a graph illustrating a diagram of how the urine flow may vary during a typical voiding process according to some implementations.
Figure 12 is a block-chart, schematically showing the components of the analytical sensor device and the external device according to some implementations.
Figure 13 is a flowchart describing a method for measuring at least one fluid/urine property according to some implementations.

### DETAILED DESCRIPTION OF CURRENTLY PREFERRED EMBODIMENTS

In the following detailed description, preferred embodiments of the invention will be described. However, it is to be understood that features of the different embodiments are exchangeable between the embodiments and may be combined in different ways, unless anything else is specifically indicated. For example, at least one sensor of a first type may be combined with at least one sensor of a different type. It may also be noted that, for the sake of clarity, the dimensions of certain components illustrated in the drawings may differ from the corresponding dimensions in real-life implementations of the invention. In the following exemplary embodiments, urine will be presented as the main example of a fluid which is analyzed with the analytical sensor device. However, it is understood that the fluid could be any fluid instead of urine, such as water, blood, oils or foodstuffs.

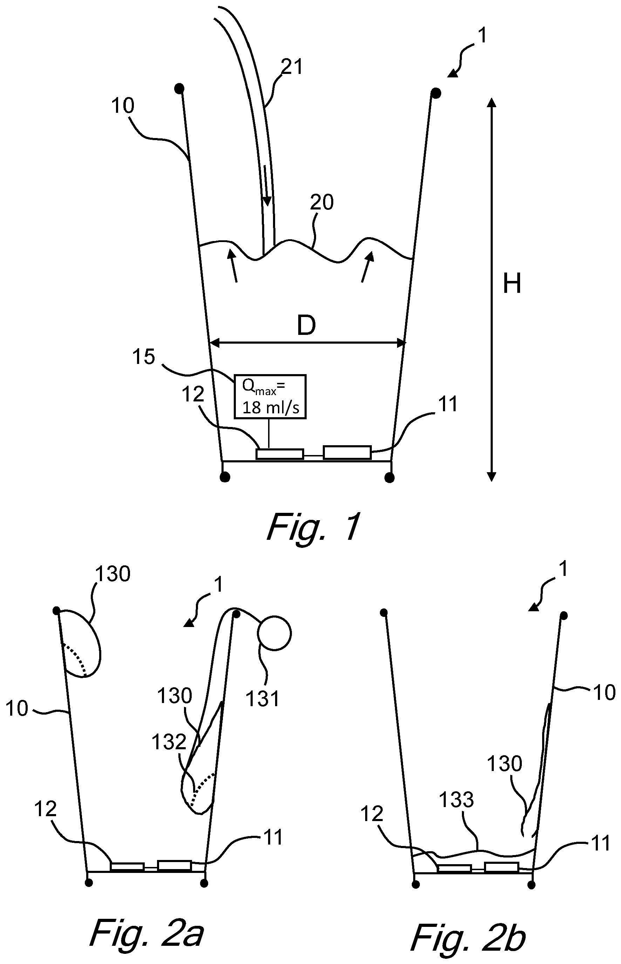

Fig. 1 shows a cross-sectional view of an analytical (medical) sensor device 1 according to some implementations. The analytical sensor device 1 is preferably fully disposable and intended for single-time use.

The analytical sensor device 1 comprises a container 10, preferably made of a fully disposable material. In the shown embodiment, the container 10 is shaped like a cup, such as a regular drinking cup. The general shape of the container 10 is adapted to be easily held by a user, such as a patient (or an assistant to the patient such as a medical professional) and/or be placed onto a flat surface. As seen, the container 10 of fig. 1 has a generally flat bottom portion with a bottom rim defining a bottom cavity, allowing it to be placed onto a flat surface. For example, the container 10 has a height H of about 50 mm to about 300 mm, and preferably about 120 mm to about 150 mm. In one embodiment, the total height of the of the container 10 is about 170 mm (including the bottom rim) and the inner height is between 150 mm and 160 mm.

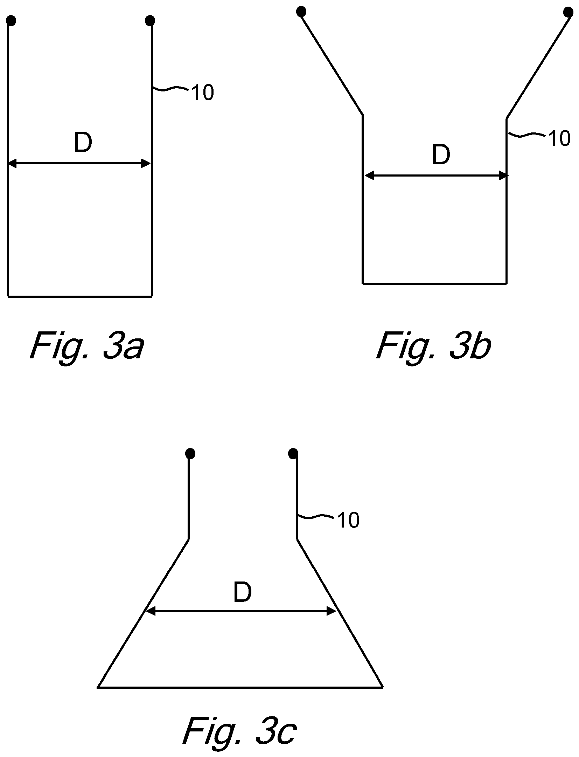

In the depicted embodiment, the diameter D increases from the bottom portion towards the open top portion. The container 10 of this embodiment is thus conical in shape. In some embodiments, the diameter of the bottom plate is between 60 mm and 80 mm, such as 70 mm, and the diameter D increases linearly to the opening which has a diameter of between 90 mm and 110 mm, such as 100 mm. An increasing diameter D has the benefit of allowing the container 10 to be easily gripped while also allowing multiple containers 10 to be stacked in a space efficient manner, allowing for more efficient transportation and/or storage of multiple analytical sensor devices 1. Other shapes of the container 10 are also envisaged and additional examples will be described in connection to figs. 3a-c.

When used for uroflowmetric measurements, the container 10 is adapted to hold at least as much urine as is expected when a patient empties her/his bladder, allowing a patient to empty her/his bladder into the container 10 without the urine 20 overflowing. For instance, the container 10 can hold at least 500 ml urine, or at least 700 ml such as at least 850 ml.

As shown in fig. 1 the analytical sensor device 1 further comprises a sensor 12 arranged inside the container 10 wherein the sensor 12 is configured to measure at least one property of urine 20, 21 which has entered the container 10. The sensor 12 is preferably configured to measure at least one of a pressure or a weight, a pH level, a temperature, an electrical property and a concentration or presence of glucose, bacteria (such as *E. coli*)*,* ammonia, sodium, potassium urea, other contents of urine 20 or a foreign substance, including but not limited to blood, chloride, sodium, potassium, creatinine or other dissolved ions and inorganic or organic compounds such as drugs or secondary metabolites of drugs.

The sensor 12 is powered by an energy source 11, wherein the energy source comprises a biofuel cell which in this embodiment is also arranged inside the container 10. Preferably, the biofuel cell is located at a bottom portion of the container 10 so as to immediately come into contact with urine 20 when a patient voids into the container 10. The biofuel cell is configured to be activated (i.e. start supplying power) when it comes into contact with an activation fluid, such as urine 20 or a limited amount of activation fluid released from an activation fluid reservoir. Thus, the analytical sensor device 1 may be in an inherently inactivated state until the patient voids into the container 10, and urine comes into contact with the energy source 11, whereby the analytical sensor device 1 is activated automatically.

The biofuel cell may be made of, or based on, a bio-based and/or biodegradable and/or bio-compostable material. Preferably, the biofuel cell is made of a bio-based material. Such material(s) may be selected from the group comprising, natural carbon materials (graphite, black carbons), natural polymers (chitin, cellulose, microcrystalline cellulose, alginate, starch, polysaccharides, natural rubber) and/or biodegradable polymers (such as xanthan gum, polylactic acid, poly caprolactone). Such materials may further be selected from natural fibers (chitosan, collagen, keratin). The biodegradable material may be substantially a cellulose fiber blend such as paper or cardboard. The biofuel cell may be an enzymatic fuel cell or a microbiologic fuel cell, preferably made from vegetable fibers, for example paper-based. Vegetable fibers or paper-based biofuel cells are advantageous due to their low cost, the flexibility allowing them to conform to various surfaces, and the low environmental impact. Examples of advantageous paper-based biofuel cells are those provided by the company BeFC (French company). Advantageously, the paper-based biofuel cell may be as disclosed in the published patent applications US 2021/0249676, WO 2021/170826 and WO 2021/094593. The biofuel cell is also preferably made of a bio-based material.

The biofuel cell is preferably free of metal and is consequently an energy source well suited for a disposable product. In an embodiment, the biofuel cell is made of bio-polymers, enzymes or bacteria and carbons. The biofuel cell may comprise a microbial and/or enzymatic solution. The biofuel cell can in such embodiments be made extremely thin, flexible and small, which makes it well adapted to be integrated in an analytical sensor device. The biofuel cell can in such embodiments be easily shaped in any form such as circle, square, rectangle, or pentagon for instance.

The biofuel cell may be arranged to use biological catalysts instead of chemical or expensive noble metal catalysts to convert natural substrates such as glucose and oxygen into electricity. The biofuel cell preferably uses biofuel enzyme cells to produce electrical energy from biological substances, such as oxygen and/or glucose, which are present in biological fluids, such as sweat, blood and urine. In some implementations, the biofuel cell comprises the biological substance (e.g. glucose) which is released when the biofuel cell comes into contact with a solvent such as water or urine or the activation fluid. In some implementations, the biological substance is added via the urine or fluid entering into the container or the biological substance is present in the activation fluid. Oxygen from the air may reach the biofuel cell and it is also envisaged that in embodiments wherein the biofuel cell is contained in a blister the blister contains oxygen.

The biofuel cell may be a single cathode cell (SC), where a cathode is positioned between an anode and a support. Alternatively, the biofuel cell may be realized as a single cathode air cell (SABC) or a double cathode air cell (DABC). In a SABC realization, the support of the SC realization may be replaced with a support which is permeable to air and allows penetration of oxygen. In a DABC realization, the biofuel cell comprises two cathodes arranged on each side of an anode, and with permeable support layers arranged on the outside of each of the two cathodes. However, other realizations are also feasible, as is per se known in the art.

In some implementations, the analytical sensor device 1 further comprises a presentation unit 15 configured to emit a light (such as a LED) and/or display a graphical message (such as an LCD display or an electronic ink display) and/or emit a sound, based on the result of the measurement performed by the sensor 12. In some implementations, the sensor 12 is a pressure sensor 12 configured to measure the fluid pressure of urine 20 over time when a urine flow 21 enters the container 10. From the change in pressure over time (and the known inner shape of the container 10) the volume of urine added per unit of time (urine flow rate) can be extracted. The average flow rate, or maximum instantaneous urine flow rate measured may be displayed by the presentation unit 15 allowing the patient and/or patient's assistant to read the result of the measurement.

Similarly, if the presentation unit emits a sound instead of, or in addition to, displaying information visually, the presentation unit 15 may emit an alarm signal if the measured property is above or below a certain threshold level. For instance, if the maximum urine flow rate Qₘₐₓ is below 10 ml/s or below 15 ml/s a visual or acoustic signal is emitted letting the patient or patient's assistant know that the urine flow 21 is low, which could mean that further urologic examination is advisable.

The analytical sensor device 1 may further comprise a controller or CPU (not shown) which is also powered by the energy source 11. The controller being configured to control at least one of the sensor 12, an optional presentation unit 15 or sound emitting device and an optional wireless transmitter (not shown) for transmitting measurement data to an external device (also not shown).

The controller or CPU may further be configured to process the sensor data so as to form measurement information. This processing may comprise converting a series of pressure or electrical property measurements into a corresponding series of urine flow rate measurements. Optionally, the controller also analyzes the measurement information and/or the sensor data so as to determine e.g. an average urine flow rate, the maximum urine flow rate or a total urine volume added to the container 10. Alternatively, the controller is only configured to temporarily store the sensor data or measurement information and control a wireless transmitter to convey the sensor data or measurement information to an external device (e.g. a smartphone or external computer) which performs the processing and/or analysis of the sensor data and/or measurement information.

It is understood that all electronic circuits of the energy source 11 and sensor 12 may be arranged inside the container 10. However, at least some of the electronic circuits may be integrated into the container 10 (such as in the walls or bottom of the container) or arranged on the outside of the container 10 (e.g. on the outside of the bottom portion) wherein the container-integrated or outside electronic circuits are connected to the inside-located parts of the electronic circuits with a small cable, conductor or similar. Specifically, at least the sensory part of the at least one sensor 12 is arranged in fluid communication with the inside of the container 10 to measure at least one property of the urine 20 or fluid.

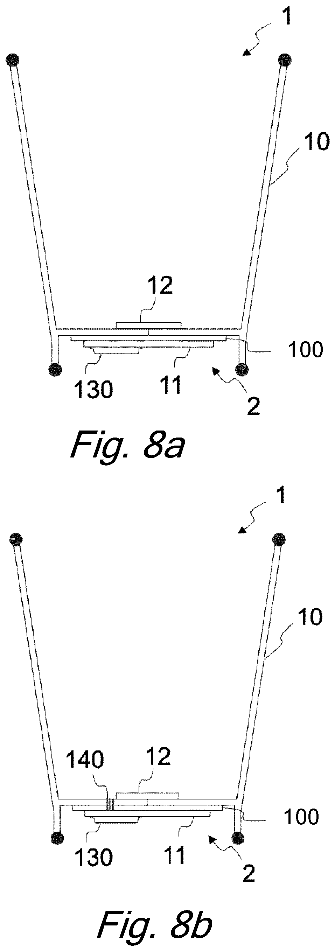

Fig. 2a depicts an analytical sensor device 1 wherein the container 10 has been provided with a fluid reservoir 130 containing an activation fluid which can activate the biofuel cell of the energy source 11. As described in the above, the biofuel cell provides power when it comes into contact with urine, however it is beneficial if the biofuel cell is activated just before the urine enters the container 10 such that the sensor 12 performs measurements throughout the patient's entire voiding process. To this end, a fluid reservoir 130 containing a fluid which can activate the biofuel cell may be arranged inside the container 10. With further reference to fig. 2b it is shown that by opening the fluid reservoir 130 the activation fluid 133 is released inside the container 10 and activates the biofuel cell of the energy source 11. Alternatively, the fluid reservoir 130 is arranged on the outside of the container 10 as will be described in connection to fig. 8a and fig. 8b below.

Preferably, the fluid reservoir 130 of this embodiment is arranged close to the opening of the container 10 allowing easy access to open the fluid reservoir 130 without the user risking damaging the energy source 11 or sensor 12 located inside the container 10. The fluid reservoir 130 is preferably rupturable or made of a material which can be teared apart, to make the release of the activation fluid 133 easy and simple to achieve. For instance, the fluid reservoir 130 is provided with a weakened seam 132 defining a tear-line along which the reservoir 130 splits open to release the activation fluid 133. The patient or patient's assistant may then tear, squeeze or compress the fluid reservoir 130 to release the activation fluid 133.

The fluid reservoir 130 may be in the form a rupturable or openable sachet or pouch.

In some implementations, an opening string 131 is attached to the fluid reservoir 130 wherein the opening string extends out of the opening of the container 10. A user may then grab and pull the opening string 131 to open the fluid reservoir 130 and release the activation fluid 133 inside the container 10 without risking coming into contact with the activation fluid 133. Although the activation fluid 133 preferably is non-hazardous, patients or patient's assistants may prefer keeping their hands dry when using the analytical sensor device 1. Additionally, the opening string 131 allows the reservoir to be placed at otherwise hard to reach spaces inside the container, such as at or near the bottom.

Another benefit with an initial addition of activation fluid 133 is that the initial splashing of a urine stream striking a dry container 10 bottom is reduced. Yet another benefit is that a predetermined amount of activation fluid 133 is released, which may be used to calibrate the at least one sensor 12. Additionally, it is noted that the amount and composition of activation fluid 133 may have to be compensated for when e.g. determining the total amount of voided urine or the pH of the urine as the activation fluid 133 will mix with the urine. However, as the amount and composition of activation fluid 133 is known, this compensation is easily implemented when processing and analyzing the sensor data. The reservoir 130 may contain a limited amount of activation fluid 133, such as between 0.1 ml and 50 ml of activation fluid, between 0.1 ml and 10 ml of activation fluid or less than 10 ml of activation fluid.

Figs. 3a-c depicts examples of different shapes envisaged for the container 10. In fig. 1 the container 10 is shaped like a regular drinking cup with a diameter D which increases from the bottom portion towards the open top portion, however this shape is merely exemplary, and many different shapes are possible, some examples are described in the following.

Fig. 3a depicts a container 10 with a diameter D which is substantially constant along the entire height of the container 10. While this makes space efficient stacking of the container more difficult, this shape has the benefit that the fluid pressure measured at some location inside the container 10 will have a simple linear relationship with the urine column height inside the container 10 which makes analysis simpler. By comparison, if urine (or any fluid) is added at a constant rate to the container from fig. 1, the fluid pressure will increase rapidly at first, but slower and slower as the fluid heigh increases which should be considered when processing the sensor data to obtain urine flow rate measurements.

Fig. 3b shows another example of a container 10 wherein the diameter D increases at a top portion along the direction from the bottom to the open top of the container 10. This container shape has the benefits of being somewhat stackable, enabling a simple relationship between fluid pressure increase and fluid height (at least initially) and having a larger opening for receiving urine from the patient. This container shape is especially suitable for female patients.

Fig. 3c shows yet another example of a container 10 according to some implementations. This container 10 has a wide base, making it very stable when placed on a flat surface, with a diameter D which decreases over at least a portion of the height of the container towards a narrow opening. While this container 10 is difficult to stack it decreases the risk of urine splashing out of the container 10. This container is especially suitable for male patients who e.g. place their penis inside the narrow opening, reducing the risk of spilling or splashing urine outside of the container.

While fig. 3a-c depicts a cross-sectional side view of containers 10 with different diameter D profiles it is understood that the cross-sectional shape of any container 10 described herein as seen along the height axis, perpendicular to the diameter D, may vary. For example, the cross-sectional shape as seen along the height axis of the container 10 may be circular, rectangular, triangular, hexagonal or any polygonal shape.

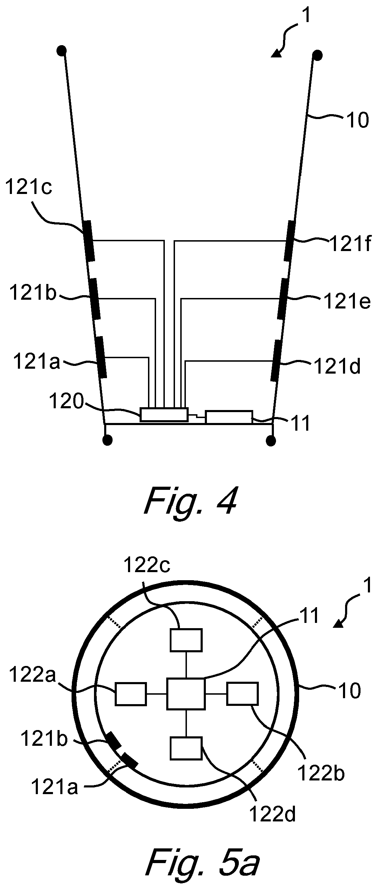

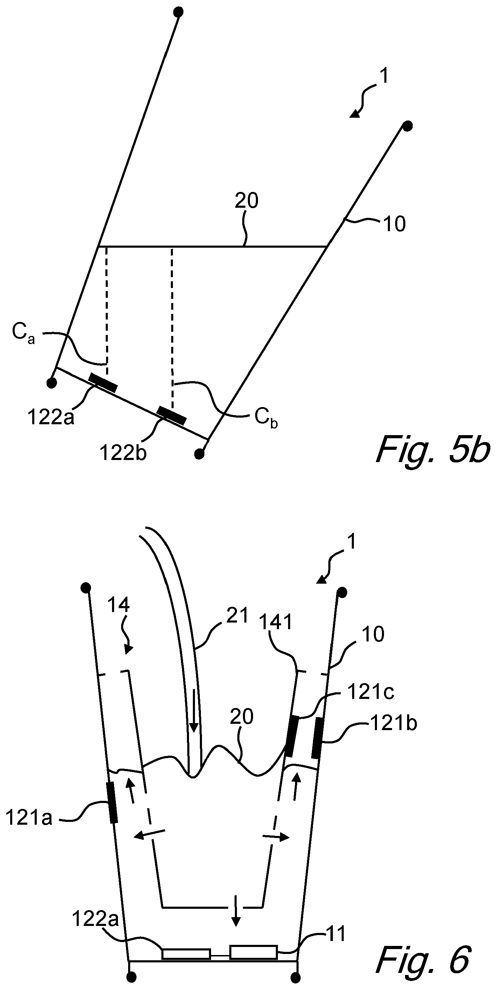

The term "sensor" is used generally to refer to a sensor measuring at least one property of the urine (or fluid) and the sensor may comprise one or more physical sensor devices, such as one or more pressure sensor and/or one or more terminals for electrical property measurements and/or one or more pH sensors. Some examples of sensors that may be placed in the container will now be described in connection to fig. 4, fig. 5a, and fig. 5b.

Fig. 4 depicts an example embodiment wherein the at least one sensor is an electrical property sensor 120 connected to at least two terminals (which may also be referred to as electrodes) 121a, 121b, 121c, 121d, 121e, 121f. Each terminal 121a, 121b, 121c, 121d, 121e, 121f is connected to the electrical sensor 120 configured to measure at least one electrical property between two of the terminals 121a, 121b, 121c, 121d, 121e, 121f. The electrical property may be an alternating current (AC) electrical property or a direct current (DC) electrical property. The electrical property is e.g. one of a capacitance, a resistance, a conductance and an inductance. The measured electrical property will indirectly indicate the amount of urine inside the container 10. In some implementations, at least one of terminals 121a, 121b, 121c, 121d, 121e, 121f is integrated with the electrical property sensor 120.

As an example, the electrical sensor 120 measures a capacitance between the two terminals 121a and 121d. The two terminals 121a and 121d act as two conductive plates and, as they are mounted to the container 10, the separation between the two terminals 121a, 121d is fixed. That is, the measured capacitance between these terminals 121a, 121d will be influenced by the electric permittivity of the media located between the terminals 121a, 121d. Accordingly, when urine reaches the terminal pairs the sensed capacitance will change, which indicates that the amount of urine has risen to reach the terminal pair 121a, 121d. It is also possible that the terminals 121a, 121b, 121c, 121d, 121e, 121f extend along the height direction of the container 10 such that when urine rises inside the container 10 the measured capacitance of each pair changes gradually as the urine covers more and more of the terminal area, allowing urine level measurements of even finer granularity to be performed even with a single pair of terminals 121a, 121b, 121c, 121d, 121e, 121f.

By placing a plurality of terminals 121a, 121b, 121c, 121d, 121e, 121f inside the container 10, e.g. in a ladder arrangement with one terminal pair above the other, and performing repeated measurements of the electrical property between pairs of terminals 121a, 121b, 121c, 121d, 121e, 121f it is possible to achieve accurate readings of the urine level over time which can easily be converted into a urine flow rate measurement indicating at what rate urine is received in the container 10.

While the above example is explained for a capacitance measurement, a similar process may be used to determine the urine level with resistive measurements. For instance, the resistance between two terminals 121a, 121d when the distance between them is filled with air is high. However, when urine reaches the terminals 121a, 121d the resistance drops as urine is a better electrical conductor than air.

Additionally, while fig. 4 illustrates that the terminals for the electrical property measurement are placed on opposite sides of the container 10 it is also envisaged that the terminals 121a, 121b, 121c, 121d, 121e, 121f may be placed much closer together, e.g. right next to each other on the same side of the container 10 or with one terminals 121a, 121b, 121c, 121d, 121e, 121f above the other. It is also envisaged that the terminals 121a, 121b, 121c and 121d, 121e, 121f, respectively may be connected together in series or in parallel. It is also envisaged that at least one terminal 121a, 121b, 121c, 121d, 121e, 121f may be placed on the bottom of the container 10 and one or more terminal(s) 121a, 121b, 121c, 121d, 121e, 121f may be arranged on the inner side walls of the container 10.

Fig. 5a shows a top view of a container 10 with another example of sensor arrangement. As seen, a plurality of pressure (or weight) sensors 122a, 122b, 122c, 122d are arranged at different locations inside the container wherein all pressure sensors 122a, 122b, 122c, 122d are powered by the energy source 11. In the shown embodiment the pressure sensors 122a, 122b, 122c, 122d are located at different locations on the bottom surface of the container 10 so as to measure the fluid pressure brought by a urine column above each sensor.

By using sensor data from two or more pressure sensors 122a, 122b, 122c, 122d the measurement accuracy can be enhanced by e.g. averaging the measured pressure of each pressure sensor 122a, 122b, 122c, 122d. Even if there is considerable splashing and waves formed inside the container 10 when urine is voided into the container 10, the usage of two or more pressure sensors 122a, 122b, 122c, 122d will provide a more stable and reliable measurement, e.g. by averaging the sensor data of all pressure sensors 122a, 122b, 122c, 122d. The same applies to using multiple terminals for electrical property measurements, whereby the electrical property between multiple terminals can be average to provide a more stable and reliable measurement.

Fig. 5a also shows two terminals 121a, 121b arranged close to each other on a side wall of the container 10. Terminals 121a, 121b (for electrical property measurements) and pressure sensors 122a, 122b, 122c, 122d may be combined in many ways to provide accurate flow rate measurements. It is understood that pressure sensors 122a, 122b, 122c, 122d may be placed at the side walls as well as at the bottom and that terminals 121a, 121b may be placed at the bottom as well as the side walls of the container 10.

As further illustrated in fig. 5b usage of two or more pressure sensors 122a, 122b also has the added benefit of enabling the sensor data from the sensors 122a, 122b to be compensated for a tilt of the container 10 during measurement. As seen, a tilt of the container 10 will entail different urine column heights Cₐ, C_{b} above each of the pressure sensors 122a, 122b. By determining the average fluid pressure using two or more sensors the effect of any tilt can be reduced. Additionally, based on the tilting angle and the known shape of the container 10 it is possible to estimate the amount of urine inside the container given at least one pressure measurement from at least one of pressure sensors 122a, 122b. To this end, while it is preferred to keep the container 10 upright and straight during urine voiding it is still possible to obtain very accurate readings even if the container 10 is tilted during filling. By adding at least a third pressure sensor, it is possible to establish and compensate for any tilt of the container 10 in three dimensions.

It is also possible to provide structural parts in the container to reduce or hinder urine from sloshing or splashing as the urine is received in the container. Such structural parts may be referred to as baffles, and some examples of such structures will now be described. The baffles may be formed as an integrated part of the container or attached to the container by an adhesive. It is also envisaged that the baffles are retained by mechanically engaging them to the container, that the baffles are flexibly resilient and retained resiliently, and/or that the baffles are retained by friction. For some types of baffles, the baffle may not be fixated to the container at all, wherein the baffle is merely placed in the container.

Fig. 6 depicts an analytical sensor device 1 wherein the container 10 has been provided with an anti-splashing or anti-sloshing baffle 14 adapted to reduce the tendency of the urine 20 to slosh around inside the container even when a concentrated urine stream 21 enters the container with a high velocity.

The baffle 14 of fig. 6 is in the shape of an insert 141 which extends along the sides of the container, the insert 141 being provided with a plurality of openings. The insert 141 defines a void space between the baffle 141 and the sides of the container 10, and optionally a void space between the baffle and the bottom of the container 10. That is, the pressure sensor 122a and terminals 121a, 121b, 121c of the analytical sensor device 1 are at least partially protected from the opening and a direct hit of the urine stream 21, while the baffle 14 still allows there to be a fluid communication between the urine stream 21 and the pressure sensors 122a and terminals 121a, 121b, 121c. That is, the baffle 14 may act as a sensor cover plate as described in further detail in the below.

In this way, as the urine 20 is forced through the openings of the baffle 14 into smaller spaces where the turbulent movement of urine is quickly absorbed by the container 10 and the baffle 14, sloshing of urine is reduced. This stabilizes the fluid surface of the urine 20 which allows for more accurate sensor measurements. This applies for all types of sensors, including pressure sensors 122a and terminals 121a, 121b, 121c for electrical property measurements.

The holes of the baffle may be of varying sizes, e.g. from 0.1 mm to 5 cm, such as between 0.1 mm and 1 cm. Alternatively, the baffle is made of a mesh, grid or screen material with very high number of openings allowing urine to flow through the baffle 14 at essentially any point on insert 141.

In some implementations, the baffle 14 produces a double wall at the inside of the container 10, with an inner wall (formed by the baffle 14) and an outer wall (formed by the side wall of the container 10). As urine enters the container 10 it can only enter the space between the inner and outer walls via an opening in the bottom and/or one or more openings provided in the inner wall. This forms a confined space suitable for placing sensors, such as terminals 121a, 121b, 121c. As seen, terminal 121c placed on the inner wall can be arranged in close proximity to the terminal 121b which facilitates accurate electrical property measurements between these two terminals 121c, 121b. Preferably, a ladder arrangement of multiple terminals 121a, 121b, 121c is placed on both the inner and outer wall of the container 10 allowing for particularly accurate electrical property measurements.

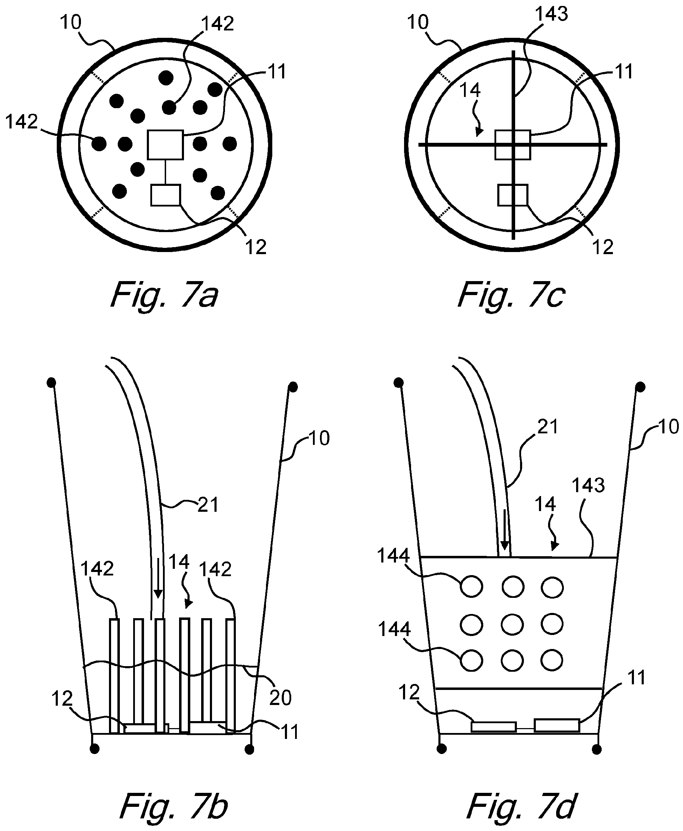

Fig. 7a and fig. 7b depicts another example of a baffle 14 for reducing splashing and sloshing of urine 20 during filling. The baffle 14 comprises a plurality of elongated rods 142, such as posts or pins arranged inside the container 10. For example, the rods extending substantially along the height of the container 10. The elongated rods 142 may have the same height, or a varying height. Preferably, the rods 142 are arranged on the bottom of the container 10 and extend substantially parallel with the direction along which the urine stream 21 is expected to enter the container 10.

The plurality of rods 142 may be attached to the container 10 or arranged on a baseplate which in turn is placed inside the container 10 prior to use. To this end, the containers 10 may still be stacked, enabling space efficient storge and transportation, with the baffle 14 being provided separately and inserted into the container 10 prior to use.

Preferably, the rods 142 and the baseplate, if present, are arranged so as to not cover the sensor 12 and the energy source 11 allowing the analytical sensor device to perform proper measurements.

In fig. 7c and 7d another example of a splash and slosh reducing baffle 14 is presented. The baffle 14 comprises one or more partitioning inserts 143 which extend across the inside of the container 10 (e.g. along at least a portion of a chord of the cross-sectional shape) to reduce splashing and sloshing. The partitioning insert(s) 143 may extend at least partially, or wholly, along the height of the container 10. Preferably, as shown in fig. 7d, the partitioning inserts 143 leaves an empty void space at the bottom of the container 10, allowing the urine 20 to come into contact with the energy source 11, optionally arranged inside the container, and sensors 12. The one or more partitioning inserts 143 may be shaped like a thin plate or barrier, e.g. made of a paper material.

In some implementations, there is one partitioning insert 143 which extends along the diagonal of the container 10. Alternatively, there are two perpendicular partitioning inserts 143 arranged in the container 10 (as shown in fig. 7c) dividing the container into four quadrants.

Additionally, the at least one partitioning insert 143 may be provided with one or more openings 144 allowing urine to flow between respective sides or quadrants of the partitioning insert(s) 143, although at a reduced rate compared to when no baffle 140 is present, which minimizes sloshing and splashing. Alternatively, the partitioning insert(s) may be perforated or made of a mesh material allowing urine to flow through the partitioning insert(s) 143 at virtual any point along their extension. A baffle 14 comprising partitioning insert(s) 143 may be made as a part of the container 10, but alternatively, the partitioning insert(s) 143 may be provided separately and inserted into the container 10 prior to use.

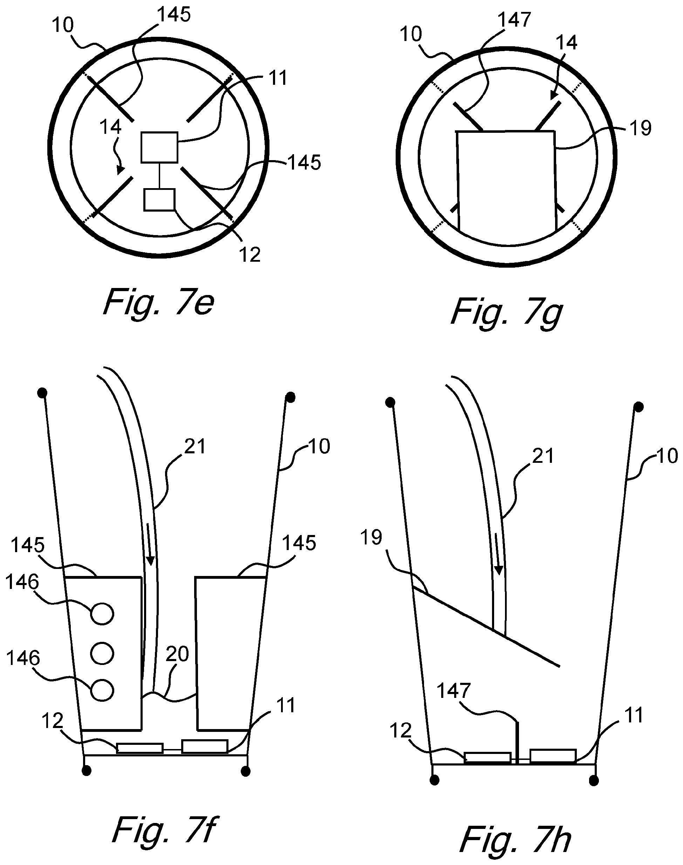

In fig. 7e and 7f yet another example of a baffle 14 for reducing urine sloshing and splashing is presented. The baffle 14 of fig. 7e and 7f comprises at least one wall segment 145 extending from an inside wall of the container 10 into the volume of the container 10. Different from the chord inserts 143, the wall segments 145 do not extend across the entire inside of the container 10. However, wall segments 145 are still effective for reducing splashing and sloshing when the container 20 is filled with urine. In the embodiments shown in fig. 7e and 7f, a plurality of wall segments 145 are present and, optionally, one or more of the wall segments 145 are provided with openings 146 to allow urine 20 to flow to the different parts of the container 10, albeit at a reduced velocity with reduced sloshing and splashing.

Fig. 7g and 7h depicts another example of a baffle 14 for reducing sloshing and splashing when a urine stream 21 enters the container 10. The baffle 14 comprises one or more bottom wall protrusions 147 mounted at the bottom of the container 10. The bottom wall protrusions 147 prevent urine sloshing by e.g. mitigating the urine from rotating around inside container 10.

In fig. 7g and 7h it is also illustrated that the analytical sensor device in some embodiments comprises a sensor cover plate 19 arranged to prevent a urine stream 21 entering through the opening, to strike at least one sensor 12 directly. Additionally, the sensor cover plate 19 may also prevent the urine stream 21 from directly striking the biofuel cell of the energy source 11. With a sensor cover plate 19 more accurate sensor measurements can be performed as the urine stream 21 is much less likely to create a high level of turbulence in the urine being in close proximity to the sensor 12. As seen, urine entering the container 10 (via the stream 21) strikes the sensor cover plate 19 and then pours or slides off the sensor cover plate 19 towards the bottom. The urine reaching the bottom will do so with much reduced velocity (compared to a direct stream hitting the bottom) which reduces sloshing and splashing. In addition, the bottom wall protrusions 147 also prevents the urine from moving in a turbulent manner close to the bottom portion. A further benefit is that the risk of the urine stream 21 damaging the sensor 12 and/or energy source 11 is greatly reduced.

The sensor cover plate 19 is preferably still small enough not to create a bottleneck for urine entering the container so as to ensure that the sensor 12 still can perform accurate readings. For example, the sensor cover plate 19 may cover 10% to 70% or 20% to 50% of the container 10 bottom when observed from the height axis of the container 10.

While fig. 7g and 7h depicts a sensor cover plate 19 in combination with bottom wall protrusions 147 acting as a baffle 14, it is noted that the sensor cover plate 19 can be combined with any of the other baffles 14 described in the above. Preferably, the sensor cover plate 19 is arranged close to the bottom of the container 10 and/or inclined so as to extend from a side wall downwards to the bottom portion, which enables space efficient stacking of multiple containers.

Additionally, the analytical sensor device may also be provided with a flange at the opening (e.g. lid with a narrow opening), a tubular receptor, a funnel or the like, to aid in directing the urine into the container and/or to avoid splashing and sloshing of urine.

Fig. 8a-b depicts an example of the analytical sensor device 1 where the energy source 11 comprising the biofuel cell, the activation fluid reservoir 130 and the electronic platform 100 are located outside of the disposable container. The analytical sensor device 1 further comprises a sensor 12 arranged inside the container 10.

Fig. 8a shows an example embodiment wherein the electronic platform 100, the biofuel cell 11, and the activation fluid reservoir 130 are placed in a cavity 2 underneath the container 10. In this embodiment, the biofuel cell of the energy source 11 is solely powered using the activation fluid contained in the fluid reservoir 130. For example, the biofuel cell of the energy source 11 and the activation fluid reservoir 130 is arranged inside an enclosed space (such as a plastic dome or sachet) wherein the activation fluid reservoir 130 is opened, allowing the activation to come into contact with and activate the biofuel cell.

Fig. 8b shows another example embodiment wherein the electronic platform 100, energy source 11, and a fluid reservoir 130 are placed in the cavity 2 underneath the container 10. In this embodiment, the biofuel cell of the energy source 11 is activated using the activation fluid contained in the fluid reservoir 130 and can then be fed and further powered with urine via channels 140 through the bottom of the container 10 and the electronic platform 100. Alternatively, the energy source 11 and the fluid reservoir 130 could be placed inside the container 10 as shown in fig. 2a and fig. 2b, whereas the electronic platform 100, transmitter or controller/CPU remains in the cavity 2 underneath the container 10.

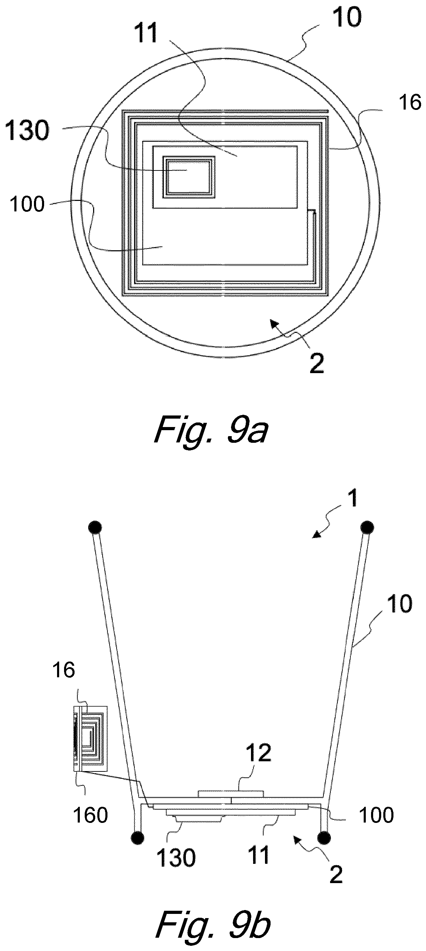

Fig. 9a-c depicts examples of the analytical sensor device 1 with different placements of the wireless transmitter 16. The wireless transmitter 16 may be an antenna.

Fig. 9a shows a bottom view of the container 10 wherein the wireless transmitter (or antenna) 16 is printed directly on the container 10 in the underneath cavity 2 and connected to the electronic platform 100. The electronic platform 100 is powered by the biofuel cell of the energy source 11 which is activated by the activation fluid in the fluid reservoir 130.

Alternatively, as shown in fig. 9b, the wireless transmitter (or antenna) 16 is provided printed on a sticker 160 that can be advantageously sticked onto the container 10, in its underneath cavity 2 or on to the external wall of the container 10, and connected to the electronic platform 100.

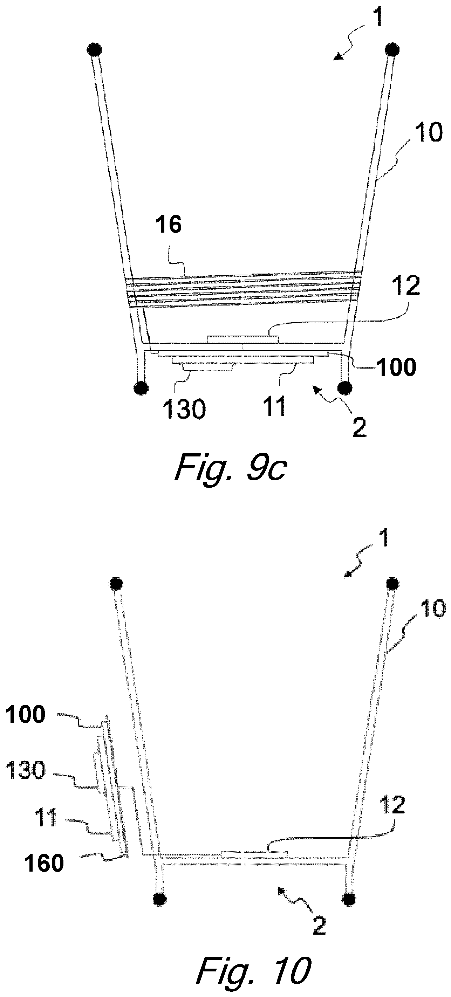

Fig. 9c depicts another exemplary embodiment wherein the wireless transmitter (or antenna) 16 is printed directly on the external wall of the container 10 and connected to the electronic platform 100. The electronic platform 100 is powered by the biofuel cell of the energy source 11 which is activated by the activation fluid in the fluid reservoir 130. The wireless transmitter 16 may e.g. circle around the outside of the container 10 multiple laps, forming e.g. a helical wireless transmitter.

Fig. 10 depicts an example of the analytical sensor device 1 where the biofuel cell of the energy source, the fluid reservoir and the electronic platform 100 are all located on a sticker. For example, these components are beforehand mounted on a sticker 160 that can be advantageously sticked on the container 10, in its underneath cavity 2 or on its external wall, and connected to the sensor 12. While the above envisaged examples of how the wireless transmitter can be arranged are advantageous, it is understood that many other options are possible. Preferably, the wireless transmitter is arranged integrated into the container 10 or arranged on the outside of the container 10 as arrangement of the wireless transmitter on the inside may degrade transmission performance due to the urine (or fluid) at least partially blocking the transmission.

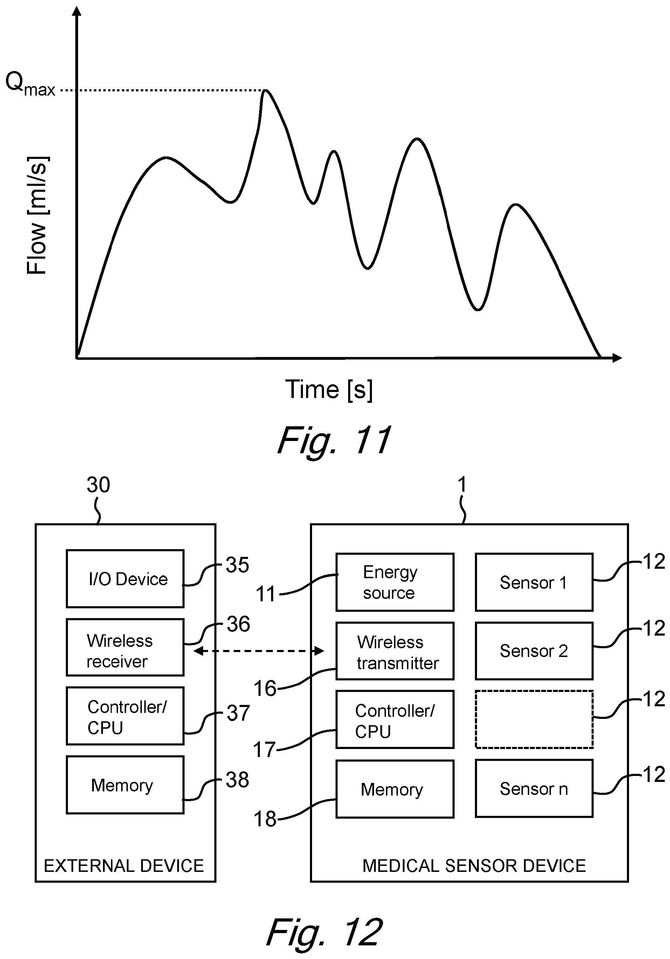

Fig. 11 is a graph showing schematically what the uroflowmetric measurement with the analytical sensor device could indicate. The horizontal axis depicts the elapsed time since measurements started (e.g. in seconds) and the vertical axis depicts the measured flow rate (e.g. expressed in milliliters per second) based on the sensor data acquired with the sensor of the analytical sensor device. As explained in the above, the sensor may measure a parameter which indirectly indicates the urine flow rate (such as a varying electrical property or pressure), however the graph of fig. 11 is the resulting flow rate as determined after processing the sensor data to form measurement information.

Sensor data is collected at different points in time, and preferably multiple times each second. For instance, new sensor data is acquired at least five times each second, at least ten times each second or at least twenty times each second. This enables the measurement information (e.g. the urine flow rate) to also be updated with a similar time-resolution. Each sensor measurement is not necessarily used to form measurement information, e.g. it is possible that the sensor data is of high resolution whereby the sensor data is down sampled, and the processing is based on a down sampled version of the sensor data.

It is common for the urine flow rate to be irregular and fluctuate during the patient's voiding process, and the graph in fig. 11 depicts such voiding process. From the graph in fig. 11, it is possible to determine many parameters which may indicate the patient is healthy or if further urologic investigation is needed. Some parameters, such as the maximum urine flow rate, Qₘₐₓ, measured during the voiding process, Qₘₐₓ, the number of oscillations or the frequency of the oscillations may be read directly from the graph or determined analytically. Some parameters are difficult to determine analytically, whereby a medical professional may review the graph and determine whether the patient appears to be healthy. For example, a flow rate that varies with large amplitude may be an indication of blockage caused by e.g. an enlarged prostate, stricture, bladder stone, meatal stenosis or stricture..

The duration of the voiding process is also easily determined from the graph and the total amount of voided urine may also be determined, e.g. by integrating the curve along the time axis (and removing the volume of any activation fluid).

Data describing the entire graph may be displayed on the analytical sensor device or transmitted for display on an external device. Additionally or alternatively, only one or more parameters such as Qₘₐₓ or the total amount of urine, is displayed on the analytical sensor device or transmitted for display on an external device. For example, the controller of the analytical sensor device may be configured to determine if Qₘₐₓ is below a predetermined threshold level (such as 10 ml/s or 15 ml/s). If this is the case the controller activates the light emitting device and/or sound emitting device to visually or acoustically make the patient or the patient's assistant aware of that Qₘₐₓ is below the predetermined threshold.

Fig. 12 is an overview of the different components provided in the analytical sensor device 1 and the optional external device 30. The analytical sensor device 1 comprises a container with an energy source 11 (comprising the biofuel cell) and one or more sensors 12 arranged on the inside of the container. The analytical sensor device may also comprise a controller or CPU 17 configured to control the sensors 12 and/or an optional light/sound emitting unit (not shown) to visually or acoustically present the result of the sensor measurements to a user. The controller or CPU 17 may be configured to process the sensor data and e.g. extract urine flow measurements from changes in electrical property or pressure and/or determine certain parameters such as Qₘₐₓ.

In some implementations, the analytical sensor device further comprises a memory module 18 (also powered by the energy source 11) allowing the controller or CPU 17 to, at least temporarily, store the sensor data while processing or analyzing the sensor data and/or store the measurement information prior to providing it to the light/sound emitting unit or a wireless transmitter 16.

As seen in fig. 12 the analytical sensor device may comprise a wireless transmitter 16 (e.g. a Bluetooth, WiFi, ZigBee or other type of transmitter) for wireless transmission of the sensor data to a wireless receiver 36 of the external device 30. As the power supplied by the energy source 11 is limited, the wireless transmitter 16 is preferably a low power transmitter suitable for short range communication.

The sensor data (or processed versions thereof) may be transmitted to the external device 30 in substantially real time, e.g. as soon as a new data point has been collected and processed, or it is envisaged that the transmission is initiated when a fixed amount of sensor data has been collected or when it is determined that the urine process has finished (e.g. when the sensor data indicate that no additional urine is added to the container). The external device 30 (being e.g. a smartphone, smartwatch or computer) may then present the received sensor data and e.g. store the measurement data in an external device memory 38.

It is envisaged that the analytical sensor device 1 performs the data processing and optionally the data analysis so as to send processed measurement information and/or analysis results to the external device 30. However, it also envisaged that the analytical sensor device 1 may only perform the data processing and not the data analysis or that the analytical sensor device 1 performs neither and transmits only the sensor raw data to the external device 30.

For instance, as power and computational power is limited in the analytical sensor device 1 (at least if the controller/CPU 17 and energy source 11 are to be kept small and simple) it is preferable to transmit the sensor raw data to the external device 30 which generally comprises a more computationally capable controller or CPU 37, whereby the external controller or CPU 37 performs the processing and/or analysis of the sensor data.

The energy source 11 is configured to supply power to any electrical components of the medical sensor device 1. For example, the energy source 11 supplies power to one or more of the wireless transmitter 16, the controller/CPU 17, the memory module 18 and the one or more sensor(s) 12.

The operation of the external device 30 may be dictated by software (e.g. in the form of an app or a program) which is downloaded to the external device 30.

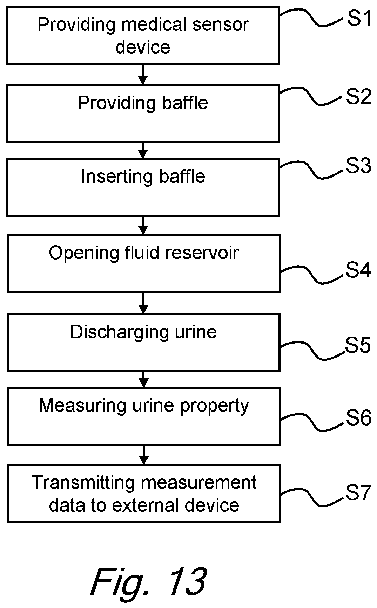

Fig. 13 is a flowchart describing a method for measuring a urine property with the analytical sensor device. At step S1, an analytical sensor device as described in the above is provided. Optionally, the method goes to step S2 comprising providing a baffle, which can be inserted into the container at step S3 to reduce splashing and sloshing of urine. Alternatively, the analytical sensor device is already provided with baffle(s) pre-installed in the container thereby rendering the steps of providing and inserting a separate baffle not needed. As a further alternative, it is possible that no baffle is used, rendering step S2 and S3 optional.

In some implementations, the analytical sensor device comprises a fluid reservoir containing an activation fluid. The method then involves step S4 of opening the fluid reservoir to release the activation fluid. When the activation fluid comes into contact with the biofuel cell of the energy source, the biofuel cell is activated.

The method then goes to step S5 involving a patient discharging urine into the container and at step S6 at least one sensor measures a property of the urine, preferably using repeated measurements during the urine discharging process.

In some implementations, the analytical sensor device comprises a presentation unit allowing the analytical sensor device to present the measurement data, or the result(s) of an analysis of the data, to a patient or patient's assistant using visual or acoustic signals. However, in some implementations the sensor data, measurement information, or analysis results are conveyed at step S7 to an external device, wherein the external device presents the sensor data, measurement data result(s) of an analysis of the data, or measurement information to the user.

The person skilled in the art realizes that the present invention by no means is limited to the preferred embodiments described above. On the contrary, many modifications and variations are possible within the scope of the appended claims. For example, the various baffle examples present in fig. 6 and fig. 7a-h may be combined with each other. Additionally, the different shapes of the container and sensor placement location inside the container described herein are merely exemplary and many additional alternatives are possible.

## Claims

1. A disposable analytical sensor device (1) comprising:
a) a disposable container (10) comprising an opening in fluid communication with an inside of the disposable container,
b) at least one sensor (12) arranged in fluid communication with the inside of said disposable container (10), the at least one sensor (12) being configured to measure at least one property of a fluid (20) present in the disposable container (10), and
c) an energy source (11) arranged on said disposable container (10) and connected to said at least one sensor (12), wherein the energy source (11) comprises a biofuel cell arranged to power the at least one sensor (12);
wherein the biofuel cell is arranged either inside the disposable container (10), preferably at a bottom portion thereof, or or on an outer surface of the disposable container (10);
and wherein the biofuel cell is located inside an enclosed space, the enclosed space comprising a fluid reservoir (130) with an activation fluid (133), the fluid reservoir (130) being configured to be opened to release the activation fluid (133) inside the enclosed space such that the activation fluid (133) comes into contact with, and activates, the biofuel cell.

2. The analytical sensor device (1) according to claim 1, wherein said at least one sensor (12) is configured to measure the flow rate of the fluid (20) entering the disposable container (10).

3. The analytical sensor device according to claim 2, wherein the at least one sensor (12) comprises:
a pressure sensor (122a, 122b, 122c, 122d), configured to measure the fluid pressure of the fluid (20) inside the disposable container (10) at different points in time, a difference in pressure between the different points in time indicating the flow rate of the fluid (20).

4. The analytical sensor device (1) according to claim 3, wherein the at least one sensor (12) comprises:
two or more pressure sensors (122a, 122b, 122c, 122d), arranged to measure the fluid pressure at two or more different locations inside the disposable container (10).

5. The analytical sensor device (1) according to any of claims 2-4, wherein the at least one sensor comprises:
an electrical sensor (120) connected to at least two electrical terminals (121a, 121b, 121c, 121d, 121e, 121f) arranged with a separation distance inside the disposable container (10), the electrical sensor (120) being configured to measure an electrical property between the at least two terminals (121a, 121b, 121c, 121d, 121e, 121f) at different points in time, a difference in electrical property between the different points in time indicating the flow rate of the fluid.

6. The analytical sensor (1) device according to any of the preceding claims, wherein said at least one sensor (12) is configured to measure at least one of
a concentration of a fluid constituent such as urea, glucose, or bacteria,
a pH level of the fluid,
a temperature of the fluid, and
a foreign substance in the fluid, such as drugs or secondary metabolites of drugs.

7. The analytical sensor device (1) according to any of the preceding claims, further comprising:
a controller (17) configured to obtain sensor data from the sensor (12) and control, based on the sensor data, a wireless transmitter (16) or a presentation unit (15) for emitting light, sound or displaying a graphical symbol of the analytical sensor device (1).

8. The analytical sensor device (1) according to any of the preceding claims, further comprising:
a wireless transmitter (16) configured to wirelessly transmit measurement information based on measurement data collected by the at least one sensor (12) to an external device (30).

9. The analytical sensor device (1) according to any one of claims 1 to 8 in which, the biofuel cell is arranged at the inside of the disposable container (10), the analytical sensor device (1) further comprises:
a fluid reservoir (130) containing an activation fluid (133) arranged on the inside of the disposable container (10), wherein said fluid reservoir (130) is configured to be opened to release the activation fluid (133) inside the disposable container (10) such that the activation fluid (133) comes into contact with, and activates, the biofuel cell.

10. The analytical sensor device (1) according to according to any one of claims 1 to 9 in which, if the biofuel cell is arranged at the inside of the disposable container (10),
the biofuel cell is configured to be powered by the liquid entering the disposable container (10).

11. The analytical sensor device (1) according to any of the preceding claims,
wherein the disposable container (10) is made of a cellulose-based material, preferably a surface-modified cellulose, a hydrophobized cellulose or a cellulose-based material provided with a non-cellulose-based layer, on at least the inside of the disposable container.

12. The analytical sensor device (1) according to any of the preceding claims,
wherein the disposable container (10) extends along a height axis, from the bottom portion to the opening, and wherein the disposable container (10) has a uniform cross-sectional shape with a fixed diameter (D) along at least a portion of the height axis.

13. The analytical sensor device (1) according to any of claims 1-11,
wherein the disposable container (10) extends along a height axis, from the bottom portion to the opening, and wherein the container has a cross-section with a diameter (D) which increases or decreases along at least a portion of the height axis.

14. The analytical sensor device (1) according to any of the preceding claims,
wherein the inside of the disposable container is provided with an anti-slosh and/or anti-splash baffle (14).

15. The analytical sensor device (1) according to any of the preceding claims, further comprising:
at least one dipstick arranged on the inside of the disposable container (10), the dipstick being configured to provide a visible indicator in response to one or more predetermined constituents being present in the fluid (20).

16. The analytical sensor device (1) according to any of the preceding claims,
wherein the fluid (20) is urine, preferably urine discharged directly from a patient into the disposable container (10).

17. An analytical sensor system comprising:
an analytical sensor device (1) according to claim 8, and
an external device (30) configured to receive and present the measurement information.

18. A method for measuring at least one property of a fluid (20), the method comprising:
providing (S1) an analytical sensor device (1) according to any of the preceding claims;
discharging (S5) fluid into the disposable container (10); and
measuring (S6) the at least one fluid property with the at least one sensor (12) of the analytical sensor device (1);
wherein the analytical sensor device (1) further comprises a fluid reservoir (130) containing an activation fluid (133), said method further comprising:
opening the fluid reservoir (130) to release the activation fluid (133) such that the activation fluid (133) comes into contact with, and activates, the biofuel cell prior to the discharging (S5) fluid into the disposable container (10).

## Patentansprüche

1. Analytische Einwegsensorvorrichtung (1), umfassend:
a) einen Einwegbehälter (10), der eine Öffnung in Fluidkommunikation mit einer Innenseite des Einwegbehälters umfasst,
b) zumindest einen Sensor (12), der in Fluidkommunikation mit der Innenseite des Einwegbehälters (10) angeordnet ist, wobei der zumindest eine Sensor (12) dazu konfiguriert ist, zumindest eine Eigenschaft eines Fluids (20), das in dem Einwegbehälter (10) vorhanden ist, zu messen, und
c) eine Energiequelle (11), die an dem Einwegbehälter (10) angeordnet und mit dem zumindest einen Sensor (12) verbunden ist, wobei die Energiequelle (11) eine Biobrennstoffzelle umfasst, die dazu angeordnet ist, den zumindest einen Sensor (12) anzutreiben;
wobei die Biobrennstoffzelle entweder innerhalb des Einwegbehälters (10), bevorzugt an einem Bodenabschnitt davon, oder an einer Außenfläche des Einwegbehälters (10) angeordnet ist; und
wobei sich die Biobrennstoffzelle innerhalb eines umschlossenen Raums befindet, wobei der umschlossene Raum ein Fluidreservoir (130) mit einem Aktivierungsfluid (133) umfasst, wobei das Fluidreservoir (130) dazu konfiguriert ist, geöffnet zu werden, um das Aktivierungsfluid (133) innerhalb des umschlossenen Raums freizugeben, sodass das Aktivierungsfluid (133) in Kontakt mit der Biobrennstoffzelle kommt und diese aktiviert.

2. Analytische Sensorvorrichtung (1) nach Anspruch 1, wobei der zumindest eine Sensor (12) dazu konfiguriert ist, die Flussrate des Fluids (20), das in den Einwegbehälter (10) eintritt, zu messen.

3. Analytische Sensorvorrichtung nach Anspruch 2, wobei der zumindest eine Sensor (12) Folgendes umfasst:
einen Drucksensor (122a, 122b, 122c, 122d), der dazu konfiguriert ist, den Fluiddruck des Fluids (20) innerhalb des Einwegbehälters (10) zu verschiedenen Zeitpunkten zu messen, wobei eine Druckdifferenz zwischen den verschiedenen Zeitpunkten die Flussrate des Fluids (20) angibt.

4. Analytische Sensorvorrichtung (1) nach Anspruch 3, wobei der zumindest eine Sensor (12) Folgendes umfasst:
zwei oder mehr Drucksensoren (122a, 122b, 122c, 122d), die dazu angeordnet sind, den Fluiddruck an zwei oder mehr verschiedenen Stellen innerhalb des Einwegbehälters (10) zu messen.

5. Analytische Sensorvorrichtung (1) nach einem der Ansprüche 2-4, wobei der zumindest eine Sensor Folgendes umfasst:
einen elektrischen Sensor (120), der mit zumindest zwei elektrischen Anschlüssen (121a, 121b, 121c, 121d, 121e, 121f) verbunden ist, die mit einem Trennabstand innerhalb des Einwegbehälters (10) angeordnet sind, wobei der elektrische Sensor (120) dazu konfiguriert ist, eine elektrische Eigenschaft zwischen den zumindest zwei Anschlüssen (121a, 121b, 121c, 121d, 121e, 121f) zu verschiedenen Zeitpunkten zu messen, wobei eine Differenz in der elektrischen Eigenschaft zwischen den verschiedenen Zeitpunkten die Flussrate des Fluids angibt.

6. Analytische Sensor(1)vorrichtung nach einem der vorhergehenden Ansprüche, wobei der zumindest eine Sensor (12) dazu konfiguriert ist, zumindest eines von Folgendem zu messen:
einer Konzentration eines Fluidbestandteils wie Harnstoff, Glukose oder Bakterien,
einem pH-Niveau des Fluids,
einer Temperatur des Fluids, und
einer Fremdsubstanz in dem Fluid, wie Arzneimittel oder sekundäre Metaboliten von Arzneimitteln.

7. Analytische Sensorvorrichtung (1) nach einem der vorhergehenden Ansprüche, ferner umfassend:
eine Steuerung (17), die dazu konfiguriert ist, Sensordaten von dem Sensor (12) zu erhalten und basierend auf den Sensordaten einen drahtlosen Sender (16) oder eine Präsentationseinheit (15) zum Emittieren von Licht, Ton oder Anzeigen eines grafischen Symbols der analytischen Sensorvorrichtung (1) zu steuern.

8. Analytische Sensorvorrichtung (1) nach einem der vorhergehenden Ansprüche, ferner umfassend:
einen drahtlosen Sender (16), der dazu konfiguriert ist, Messinformationen basierend auf Messdaten, die durch den zumindest einen Sensor (12) gesammelt werden, drahtlos an eine externe Vorrichtung (30) zu übertragen.

9. Analytische Sensorvorrichtung (1) nach einem der Ansprüche 1 bis 8, in der, wenn die Biobrennstoffzelle an der Innenseite des Einwegbehälters (10) angeordnet ist, die analytische Sensorvorrichtung (1) ferner Folgendes umfasst:
ein Fluidreservoir (130), das ein Aktivierungsfluid (133) enthält, das an der Innenseite des Einwegbehälters (10) angeordnet ist, wobei das Fluidreservoir (130) dazu konfiguriert ist, geöffnet zu werden, um das Aktivierungsfluid (133) innerhalb des Einwegbehälters (10) freizugeben, sodass das Aktivierungsfluid (133) in Kontakt mit der Biobrennstoffzelle kommt und diese aktiviert.

10. Analytische Sensorvorrichtung (1) nach nach einem der Ansprüche 1 bis 9, in der, wenn die Biobrennstoffzelle an der Innenseite des Einwegbehälters (10) angeordnet ist, die Biobrennstoffzelle dazu konfiguriert ist, durch die Flüssigkeit, die in den Einwegbehälter (10) eintritt, angetrieben zu werden.

11. Analytische Sensorvorrichtung (1) nach einem der vorhergehenden Ansprüche, wobei der Einwegbehälter (10) aus einem cellulosebasierten Material, bevorzugt einer oberflächenmodifizierten Cellulose, einer hydrophobierten Cellulose oder einem cellulosebasierten Material, das mit einer nichtcellulosebasierten Schicht bereitgestellt ist, auf zumindest der Innenseite des Einwegbehälters hergestellt ist.

12. Analytische Sensorvorrichtung (1) nach einem der vorhergehenden Ansprüche, wobei sich der Einwegbehälter (10) entlang einer Höhenachse von dem Bodenabschnitt zu der Öffnung erstreckt und wobei der Einwegbehälter (10) eine gleichmäßige Querschnittsform mit einem festen Durchmesser (D) entlang zumindest eines Abschnittes der Höhenachse aufweist.

13. Analytische Sensorvorrichtung (1) nach einem der Ansprüche 1-11,
wobei sich der Einwegbehälter (10) entlang einer Höhenachse von dem Bodenabschnitt zu der Öffnung erstreckt und wobei der Behälter einen Querschnitt mit einem Durchmesser (D) aufweist, der entlang zumindest eines Abschnittes der Höhenachse zunimmt oder abnimmt.

14. Analytische Sensorvorrichtung (1) nach einem der vorhergehenden Ansprüche,
wobei die Innenseite des Einwegbehälters mit einer Antischwapp- und/oder Antispritz-Ablenkplatte (14) bereitgestellt ist.

15. Analytische Sensorvorrichtung (1) nach einem der vorhergehenden Ansprüche, ferner umfassend:
zumindest einen Messstab, der an der Innenseite des Einwegbehälters (10) angeordnet ist, wobei der Messstab dazu konfiguriert ist, als Reaktion darauf, dass ein oder mehrere vorbestimmte Bestandteile in dem Fluid (20) vorhanden sind, einen sichtbaren Indikator bereitzustellen.

16. Analytische Sensorvorrichtung (1) nach einem der vorhergehenden Ansprüche, wobei das Fluid (20) Urin ist, bevorzugt Urin, der direkt von einem Patienten in den Einwegbehälter (10) abgegeben wird.

17. Analytisches Sensorsystem, umfassend:
eine analytische Sensorvorrichtung (1) nach Anspruch 8, und
eine externe Vorrichtung (30), die dazu konfiguriert ist, die Messinformationen zu empfangen und zu präsentieren.

18. Verfahren zum Messen von zumindest einer Eigenschaft eines Fluids (20), wobei das Verfahren Folgendes umfasst:
Bereitstellen (S1) einer analytischen Sensorvorrichtung (1) nach einem der vorhergehenden Ansprüche;
Abgeben (S5) von Fluid in den Einwegbehälter (10); und
Messen (S6) der zumindest einen Fluideigenschaft mit dem zumindest einen Sensor (12) der analytischen Sensorvorrichtung (1);
wobei die analytische Sensorvorrichtung (1) ferner ein Fluidreservoir (130) umfasst, das ein Aktivierungsfluid (133) enthält, wobei das Verfahren ferner Folgendes umfasst:
Öffnen des Fluidreservoirs (130), um das Aktivierungsfluid (133) freizugeben, sodass das Aktivierungsfluid (133) vor dem Abgeben (S5) von Fluid in den Einwegbehälter (10) in Kontakt mit der Biobrennstoffzelle kommt und diese aktiviert.

## Revendications

1. Dispositif de capteur analytique jetable (1), comprenant :
a) un récipient jetable (10) comprenant une ouverture en communication fluidique avec un intérieur du récipient jetable,
b) au moins un capteur (12) agencé en communication fluidique avec l'intérieur dudit récipient jetable (10), l'au moins un capteur (12) étant configuré pour mesurer au moins une propriété d'un fluide (20) présent dans le récipient jetable (10), et
c) une source d'énergie (11) agencée sur ledit récipient jetable (10) et reliée audit au moins un capteur (12), ladite source d'énergie (11) comprenant une pile à biocombustible agencée pour alimenter l'au moins un capteur (12) ;
dans lequel la pile à biocombustible est agencée soit à l'intérieur du récipient jetable (10), de préférence au niveau d'une partie inférieure de celui-ci, soit sur une surface extérieure du récipient jetable (10) ;
et dans lequel la pile à biocombustible est située à l'intérieur d'un espace clos, l'espace clos comprenant un réservoir de fluide (130) contenant un fluide d'activation (133), le réservoir de fluide (130) étant conçu pour être ouvert afin de libérer le fluide d'activation (133) à l'intérieur de l'espace clos de sorte que le fluide d'activation (133) entre en contact avec, et active, la pile à biocombustible.

2. Dispositif de capteur analytique (1) selon la revendication 1, dans lequel ledit au moins un capteur (12) est configuré pour mesurer le débit du fluide (20) entrant dans le récipient jetable (10).

3. Dispositif de capteur analytique selon la revendication 2, dans lequel l'au moins un capteur (12) comprend :
un capteur de pression (122a, 122b, 122c, 122d), configuré pour mesurer la pression de fluide du fluide (20) à l'intérieur du récipient jetable (10) à différents instants, une différence de pression entre les différents instants indiquant le débit du fluide (20).

4. Dispositif de capteur analytique (1) selon la revendication 3, dans lequel l'au moins un capteur (12) comprend :
deux capteurs de pression (122a, 122b, 122c, 122d) ou plus, agencés pour mesurer la pression de fluide à deux emplacements différents ou plus à l'intérieur du récipient jetable (10).

5. Dispositif de capteur analytique (1) selon l'une quelconque des revendications 2 à 4, dans lequel l'au moins un capteur comprend :
un capteur électrique (120) relié à au moins deux bornes électriques (121a, 121b, 121c, 121d, 121e, 121f) agencées à une distance de séparation à l'intérieur du récipient jetable (10), le capteur électrique (120) étant configuré pour mesurer une propriété électrique entre les au moins deux bornes (121a, 121b, 121c, 121d, 121e, 121f) à différents instants, une différence de propriété électrique entre les différents instants indiquant le débit du fluide.

6. Dispositif de capteur (1) analytique selon l'une quelconque des revendications précédentes, dans lequel ledit au moins un capteur (12) est configuré pour mesurer au moins l'un
d'une concentration d'un constituant de fluide tel que l'urée, le glucose ou des bactéries,
d'un niveau de pH du fluide,
d'une température du fluide, et
d'une substance étrangère dans le fluide, telle que des médicaments ou des métabolites secondaires de médicaments.

7. Dispositif de capteur analytique (1) selon l'une quelconque des revendications précédentes, comprenant en outre :
un dispositif de commande (17) configuré pour obtenir des données de capteur à partir du capteur (12) et commander, sur la base des données de capteur, un émetteur sans fil (16) ou une unité de présentation (15) pour émettre de la lumière, du son ou afficher un symbole graphique du dispositif de capteur analytique (1).

8. Dispositif de capteur analytique (1) selon l'une quelconque des revendications précédentes, comprenant en outre :
un émetteur sans fil (16) configuré pour transmettre sans fil des informations de mesure sur la base de données de mesure collectées par l'au moins un capteur (12) à un dispositif externe (30).

9. Dispositif de capteur analytique (1) selon l'une quelconque des revendications 1 à 8, dans lequel, si la pile à biocombustible est agencée à l'intérieur du récipient jetable (10), le dispositif de capteur analytique (1) comprend en outre :
un réservoir de fluide (130) contenant un fluide d'activation (133) agencé à l'intérieur du récipient jetable (10), ledit réservoir de fluide (130) étant configuré pour être ouvert afin de libérer le fluide d'activation (133) à l'intérieur du récipient jetable (10) de sorte que le fluide d'activation (133) entre en contact avec, et active, la pile à biocombustible.

10. Dispositif de capteur analytique (1) selon selon l'une quelconque des revendications 1 à 9 dans lequel, si la pile à biocombustible est agencée à l'intérieur du récipient jetable (10), la pile à biocombustible est configurée pour être alimentée par le liquide entrant dans le récipient jetable (10).

11. Dispositif de capteur analytique (1) selon l'une quelconque des revendications précédentes, dans lequel le récipient jetable (10) est constitué d'un matériau à base de cellulose, de préférence une cellulose à surface modifiée, une cellulose hydrophobisée ou un matériau à base de cellulose doté d'une couche non cellulosique, sur au moins l'intérieur du récipient jetable.

12. Dispositif de capteur analytique (1) selon l'une quelconque des revendications précédentes, dans lequel le récipient jetable (10) s'étend le long d'un axe de hauteur, de la partie inférieure à l'ouverture, et dans lequel le récipient jetable (10) présente une forme de section transversale uniforme avec un diamètre fixe (D) le long d'au moins une partie de l'axe de hauteur.

13. Dispositif de capteur analytique (1) selon l'une quelconque des revendications 1 à 11,
dans lequel le récipient jetable (10) s'étend le long d'un axe de hauteur, de la partie inférieure à l'ouverture, et dans lequel le récipient présente une section transversale avec un diamètre (D) qui augmente ou diminue le long d'au moins une partie de l'axe de hauteur.

14. Dispositif de capteur analytique (1) selon l'une quelconque des revendications précédentes, dans lequel l'intérieur du récipient jetable est doté d'une chicane antiballottement et/ou anti-éclaboussures (14).

15. Dispositif de capteur analytique (1) selon l'une quelconque des revendications précédentes, comprenant en outre :
au moins une bandelette agencée sur l'intérieur du récipient jetable (10), la bandelette étant configurée pour fournir un indicateur visible en réponse à la présence d'un ou plusieurs constituants prédéterminés dans le fluide (20).

16. Dispositif de capteur analytique (1) selon l'une quelconque des revendications précédentes, dans lequel le fluide (20) est de l'urine, de préférence de l'urine évacuée directement par un patient dans le récipient jetable (10).

17. Système de capteur analytique comprenant :
un dispositif de capteur analytique (1) selon la revendication 8, et
un dispositif externe (30) configuré pour recevoir et présenter les informations de mesure.

18. Procédé permettant la mesure d'au moins une propriété d'un fluide (20), le procédé comprenant :
la fourniture (S1) d'un dispositif de capteur analytique (1) selon l'une quelconque des revendications précédentes ;
l'évacuation (S5) d'un fluide dans le récipient jetable (10) ; et
la mesure (S6) de l'au moins une propriété de fluide avec l'au moins un capteur (12) du dispositif de capteur analytique (1) ;
dans lequel le dispositif de capteur analytique (1) comprend en outre un réservoir de fluide (130) contenant un fluide d'activation (133), ledit procédé comprenant en outre :
l'ouverture du réservoir de fluide (130) pour libérer le fluide d'activation (133) de sorte que le fluide d'activation (133) vienne en contact avec, et active, la pile à biocombustible avant l'évacuation (S5) d'un fluide dans le récipient jetable (10).
